Europäisches Patentamt

European Patent Office

Office européen des brevets

(11) Publication number: **0 433 053 A1**

## (12) EUROPEAN PATENT APPLICATION

(21) Application number: **90313540.8**

(22) Date of filing: **12.12.90**

(51) Int. Cl.⁵: **C12Q 1/22, A61L 2/26**

(30) Priority: **14.12.89 US 450394**

(43) Date of publication of application:
**19.06.91 Bulletin 91/25**

(84) Designated Contracting States:
**AT BE CH DE DK ES FR GB GR IT LI LU NL SE**

(71) Applicant: **MEDICAL COLLEGE OF OHIO**
**3000 Arlington Avenue**
**Toledo, Ohio 43614(US)**

(72) Inventor: **Hageage, George J.**
**2700 River View Drive**
**Maumee, Ohio 43537(US)**
Inventor: **Burnham, Jeffrey C.**
**638 Miami Manor**
**Maumee, Ohio 43537(US)**

(74) Representative: **West, Alan Harry et al**
**R.G.C. Jenkins & Co. 26 Caxton Street**
**London SW1H 0RJ(GB)**

(54) **Rapid biological sterility detection method and apparatus therefor.**

(57) The present invention relates to a method and apparatus for rapidly determining the effectiveness of a sterilization process. A test sample comprising a known quantity of viable sporulated microorganisms is placed into a sterilization chamber along with objects to be sterilized. After completion of the sterilization process, the test sample is removed along with the objects being sterilized. The test sample is exposed to a known quantity of a protein nutrient medium which supports the germination and growth of any remaining viable microorganisms. The microorganisms and nutrient medium are sealed aseptically within a containing means and are incubated for a predetermined period of time to promote the germination and growth of the remaining viable microorganisms. Thereafter, an indicator agent capable of undergoing a detectable change in response to the germination and growth of the microorganisms is introduced into the containing means. The indicator agent detects the presence of end products of the protease enzyme reactions produced by the germinating and growing microorganisms. Thereafter, the containing means is examined for the detectable color change indicating microorganism viability and thus insufficient sterilization.

# RAPID BIOLOGICAL STERILITY DETECTION METHOD AND APPARATUS THEREFOR

The present invention relates to a method and apparatus for rapidly determining the effectiveness of a sterilization process.

In the health care industry it is necessary to monitor the effectiveness of the sterilization processes used to sterilize medical equipment and other nondisposable articles. It is a standard practice in hospitals to include a sterility indicator in the batch of articles to be sterilized. The use of sterility indicators allows a direct and sensitive approach to assay the lethality of the sterilization process.

A standard type of sterility indicator includes a known quantity of test microbial spores which are exposed to the sterilization process. The test microorganisms are then incubated for a specified period of time and examined for possible growth of any surviving microorganisms in order to determine whether the sterilization process was sufficient to destroy the microorganisms.

A major drawback of presently available sterility indicators is the time delay in obtaining results of the sterility test. The currently available sterility indicators normally require that the microorganisms be cultured for at least two and often up to seven days in order to assure adequate detection of any surviving microorganisms. Since many health care facilities have limited resources and will reuse their "sterilized" instruments within 24-48 hours, the seven day holding period for sterility verification becomes impractical and inefficient. The FDA Center for Devices and Radiological Health (NCDRH) does permit incubations of less than seven days for new medical devices used by health care facilities, provided that the sterility indicator manufacturer validate the shorter incubation parameters in the labeling of the sterility indicator. For NCDRH validation, more than 97% of the number of individual sterility indicator tests must read positive (indicate survivors) in each partial cycle (incomplete sterilization) for the proposed incubation time to be acceptable. Using these guidelines it has not previously been possible for manufacturers to achieve a reduction in the holding period to less than 2 days.

In addition to the time delays, the use of the sterility indicator requires trained technicians and clean room facilities. Often it is necessary for the highly skilled laboratory technicians to transfer the test microorganisms from the sterility indicator to the incubating medium and to thereafter use their trained eye to check the sterility indicator for possible growth of microorganisms. It has been found that, despite the use of trained technicians and other such precautions, on occasion the tests produce false positive results due to human error or contaminated clean room facilities. As such, the test is considered positive and the articles must be resterilized which in turn causes further delays and increased costs.

In addition, certain industry standards must be followed to insure the effectiveness of the sterility indicator. These standards relate to the sensitivity and form (i.e. spore) of the microorganism employed for the specific sterilization process, product uniformity to assure consistent performance from one lot to the next, and that the challenge to the sterilization process exceeds the challenge of the natural bioburden (number of microorganisms on or in the sterility indicator) when the biological indicator is used within its performance characteristics. In order to meet these industry standards, the presently available sterility indicators require complicated handling techniques. Further, these indicators require lengthy incubation periods.

It is accordingly an objective of the present invention to avoid and overcome the foregoing and other difficulties of the prior art practices.

It is an objective of the present invention to provide a rapid biological detection method and apparatus for the absolute determination of microbial sterility following steam or ethylene oxide treatment of medical articles.

It is a further objective of the present invention to provide a rapid detection method and apparatus that work within an overnight time period (16 hours) or a one shift time period (8 hours).

It is a further objective of the present invention to provide a method and apparatus which are simple to use so that personnel with minimal training can achieve reliable results.

It is a further objective of the present invention to provide a method and apparatus which comply with FDA standards for biological sterility indicators.

It is a further objective of the present invention to provide a method and apparatus which measure extremely low population levels of Bacillus spores, i.e., down to 1 viable organism per sterility test.

These and other objectives of the present invention will be apparent from the following description and claims.

The present invention relates to a rapid biological detection method and apparatus that allows verification of the sterility of a sterilization process. The present invention provides a method and apparatus for expediting sterility verification by providing for completion of the test results within a short period of time, often within one personnel shift time period (8 hours). The present invention allows for the utilization of personnel with minimal training while still achieving reliable results.

The present invention relates in particular to a rapid and ultrasensitive method and apparatus for the measurement of enzyme release which occurs during the germination and growth process of surviving microorgamisms. In a preferred method of the invention a protease enzyme is detected by measuring exposed amino groups.

According to the present invention a test sample is placed into a sterilization chamber along with objects to be sterilized. The test sample is a known quantity of viable sporulated microorganisms contained within a vial or glassine envelope. Any known type of test sample such as the commercially available Bacillus stearothermophilus spore strips or such similar indicators can be used. After completion of the sterilization process, the test sample is removed from the chamber along with the objects being sterilized. The test sample is introduced into a known quantity of a nutrient medium. The nutrient medium supports the germination of spores and growth of any viable microorganisms. The microorganisms and nutrient medium are then sealed within a containing means. The microorganisms are then incubated for a predetermined period of time in order to promote the germination and growth of the microorganisms. In a preferred embodiment, the microorganisms are germinated for a period of time of any where from about 6 to about 24 hours. Thereafter, an indicator agent capable of undergoing a detectable visual change in response to the germination of the microorganisms is introduced into the containing means. The indicator agent detects the presence of an end product of the protease enzymes produced by the germinating spores and subsequently growing vegetative microorganisms. The preferred method of the present invention utilizes a ninhydrin assay as the detecting indicator agent in order to measure increased amino groups liberated as a result of proteolysis of the protease enzymes. The ninhydrin reacts with the free amino groups of the end products of the protease enzyme reaction thus producing a blue color that can be judged visually or quantitated on a spectrophotometer at 520nm.

Fig. 1 shows the ninhydrin reaction with decreasing concentrations of heat-shocked Bacillus stearothermophilus spores in suspension in TSB (tryptic soy broth) and TSB + Casein after 20 hours incubation.

Fig. 2 shows comparative viability assay response times for adequate versus inadequate autoclaving wherein the spore strips contain about $10^5$ microorganisms/test using ninhydrin as the indicator agent. Heat activation of the spores is accomplished with 5 minutes of autoclaving.

Fig. 3 shows comparative color response curves for autoclaved (5 min.-activated; 12 min.-inadequate; 20 min.-normal) spores contained on cellulose strips ($10^5$/strip) using a 0.35% ninhydrin indicator.

Fig. 4 shows comparative color response times for autoclaved spores in suspension ($10^5$/test) using a 0.35% ninhydrin indicator.

Fig. 5 shows two rapid color response curves for heat shocked spores ($10^5$) contained on cellulose strips.

Fig. 6 shows ninhydrin intensity (blue color development) at 15.5 and 24 hours with cellulose strips inoculated with decreasing concentrations of Bacillus stearothermophilus spores.

Fig. 7 shows the effect of varying the ninhydrin concentration on the color development when a standard concentration of amino acid glycine is used as an amino acid source.

Fig. 8 is a schematic diagram of one embodiment of an apparatus for rapidly detecting biological sterility.

Fig. 9 is a schematic diagram of one embodiment of a method for rapidly detecting biological sterility.

Fig. 10 is a schematic diagram of another embodiment of a method for rapidly detecting biological sterility.

The present invention relates to a method and apparatus for rapidly testing and monitoring the efficacy of a sterilization process. The present invention overcomes the disadvantages of the above-described methods for testing the efficacy of a sterilization process, particularly the long time period necessary to obtain an indication of bacterial viability. The present invention is especially useful with such sterilization processes as exposure to dry heat, steam or gaseous agents, for example, ethylene oxide or propylene oxide.

In accordance with the present invention it is preferred to utilize microorganisms which may be killed by the sterilization process but which show significant resistance to this process. The term "microorganisms" refers to bacteria, fungi, yeast, protozoa algae, viruses and protozoa. Bacterial spores are very resistant to heat and chemicals; much more so than vegetative bacterial cells, therefore the spores are often used to monitor sterilization procedures. A preferred organism selected for monitoring steam sterilization is Bacillus stearothermophilus spores, while for monitoring dry heat and ethylene oxide sterilization spores of certain strains of Bacillus subtilis are also used.

The spores represent a resting stage in the life cycle of the Bacillus genus. The resting spore contains a large number of active enzymes which allow the transformation from dormant cell to vegetative cell. The germination process, or the return to the vegetative state, has been described as a time-ordered sequence involving activation, triggering, initiation, and outgrowth. Activation is reversible and involves an increase in the rate and extent of germination. Triggering is irreversible and is the result of spore contact with the germinant. Initiation involves the loss of heat resistance, release of dipicolinic acid and calcium, loss of refractility and absorbance. Outgrowth results in formation of the vegetative cell.

Approximately 20% of the protein of dormant spores of various Bacillus species is degraded to amino acids early in spore germination. These amino acids are essential for rapid protein syntheses early in the germination process and in the outgrowth process. The proteins degraded are a group of low molecular weight (small) acid soluble species (SASP) and are found in the spore coat.

Spores contain a protease enzyme which is active on the major SASP and which is not found in other stages of growth. This enzyme, called spore protease is the enzyme responsible for initiating degradation of the major SASP in vivo during germination. In addition another protease enzyme (an endoprotease) capable of degrading large proteins is found in the spore.

In accordance with the present invention a test sample comprising at least one sterility test strip is utilized (which is also herein referred to as a spore strip). While reference is made to "spores" as a test microorganism it should be understood that microorganisms other than spore formers may be used in conjunction with the present invention. The spore strips utilized with the present invention are preferably constructed of a material which is inert to the microorganisms. A variety of commercial spore strips are readily available and can be utilized with the present invention. In addition, the spore strip can contain more than one type of microorganisms.

The present invention provides verification of the sterilization process within at least 6 to 16 hours. Due to the time period presently needed for incubation of the available commercial sterility indicators, the present invention greatly contributes to the overall efficiency and safety of the operation of health care facilities by proving product sterility before utilization of the product. The present invention detects the germination process of surviving spores which involves the conversion of a dormant spore (resistant phase) into a vegetative cell (labile phase).

According to the present invention, a predetermined quantity of a test sample comprising a known quantity of microorganisms is exposed to a sterilizing environment. Upon completion of the sterilization process, the test sample is removed from the sterilizing environment and is introduced into an appropriate culture medium in an aseptic container. The culture medium contains a protein substrate for the proteases liberated during spore germination and during subsequent microbial growth. The culture medium preferably comprises an aqueous solution or suspension of nutrient components (including the protein substrate) needed in order to promote the growth of viable microorganisms that may exist after the sterilization process. One example of a suitable culture medium is a protein containing microbiological broth such as tryptic soy broth (TSB) and/or TSB with specific protein additives, such as, for example, casein. Formulations for culture media are well-known to those familiar with the state of the art. The container is sealed such that the integrity of the container is maintained during an incubation period. The container is then placed into an incubator or other constant temperature environment in order to enhance the growth of any surviving microorganisms. Any microorganisms not killed during the sterilization process begin to germinate and grow during the incubation period, thus releasing active proteases which liberate detectable amino groups.

After a suitable incubation period an indicator agent for detecting amino groups or protein hydrolysates is introduced into the container, and the container is examined to determine whether growth of the microorganisms has taken place.

The indicator agent detects the presence of the end products of the protease enzyme reaction, and specifically, detects the presence of amino groups and/or protein hydrolysates, released by the germinating spores and growing vegetative cells. The release of such protease enzyme occurs early in the germination process and through the growth cycle. Therefore, the present invention is useful for rapidly detecting the presence of any surviving microorganisms.

The introduction of the indicator agent produces a change in color if the detectable amino groups are present. It is preferred that the color change be observed through transparent or translucent walls of the container. Any color change detected is an indication to an observer that the sterilization cycle had not killed all of the microorganisms and was thus insufficient to assure sterilization of the other articles during the sterilization process. An absence of a color change thus indicates that the sterilization process has killed all of the microorganisms.

In a preferred embodiment of the present invention the method of rapidly detecting the germination and growth of microorganisms utilizes ninhydrin as the indicator agent. The ninhydrin reacts with amino acids or amino compounds containing amino groups produced by the protease of the germinating and growing microorganisms. The ninhydrin provides for a colorimetric determination of the presence of amino acids, peptides or proteins by allowing measurement of the intensity of the blue color formed. The preferred concentrations of ninhydrin range from about 0.2% to 1.0% ninhydrin in its organic solvent (acetone/ethanol/ butanol).

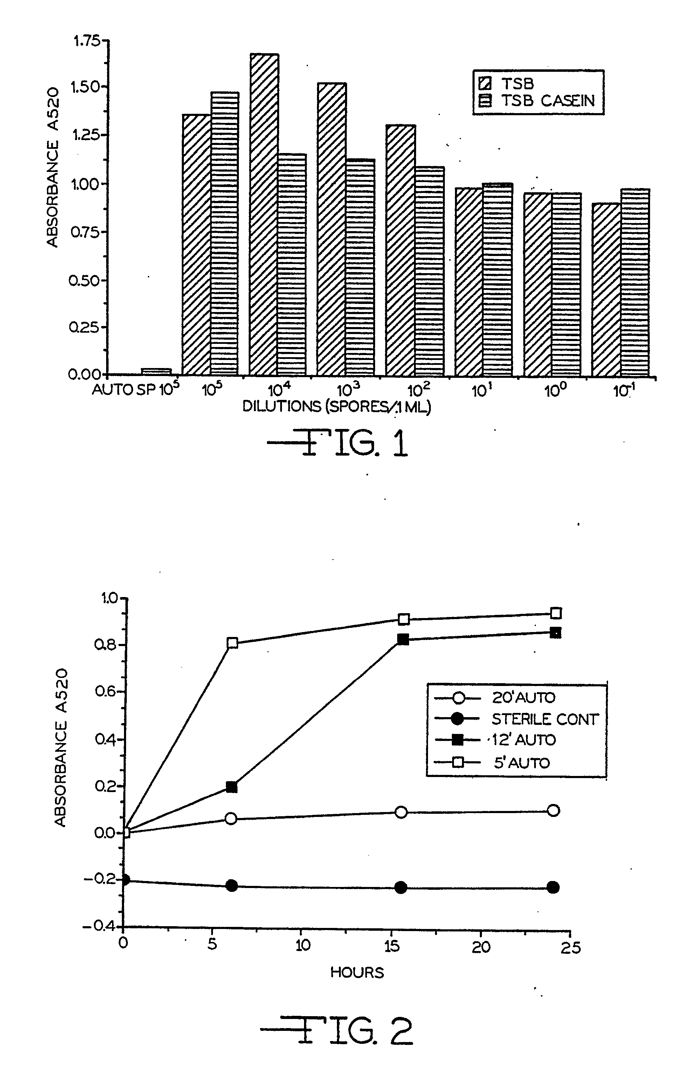

Referring now to the Figures, it is shown herein that measurement of protease enzyme activity using ninhydrin as the indicator agent is an especially rapid method to determine spore viability. Fig. 1 shows the ninhydrin reaction with a heat-shocked Bacillus stearothermophilus spore suspension in TSB and TSB plus casein after 20 hours of incubation. As shown in Fig. 1, the ninhydrin accurately detects proteolytic activity at 20 hours of growth. As can be seen, the ninhydrin detects proteolytic activity in tests which contained as few as 1 initial spore per test.

Fig. 2 shows the comparative viability assay response times for adequate versus inadequate autoclaving wherein the spore strips contained approximately $10^5$ Bacillus stearothermophilus per test. The practicability of the ninhydrin detection method of the present invention is shown by the determination of spore viability of surviving Bacillus stearothermophilus microorganisms as quickly as 6 hours after inadequate sterilization as tested by 12 minute autoclaving. No color development was seen with the normally autoclaved material (20 min.) or in the special control containing formaldehyde killed spores.

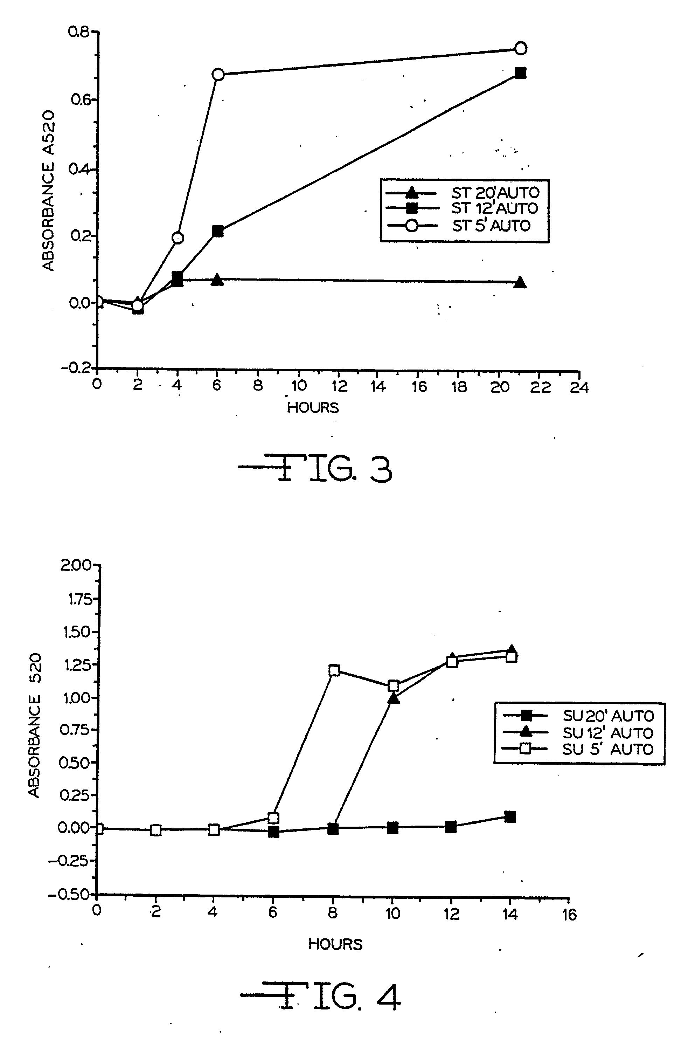

In Fig. 3 the color development can again be seen to be detectable after only 6 hours of incubation. By 24 hours the inadequately autoclaved sample has developed an equivalent amount of color as the heat activated control spores and appears a dark blue in the reaction tube.

Fig. 4 shows that spores suspended directly in the protein containing medium did not respond as quickly as the spores contained on cellulose strips. The inadequately autoclaved sample showed a two hour additional delay when the spores were prepared in this suspended manner.

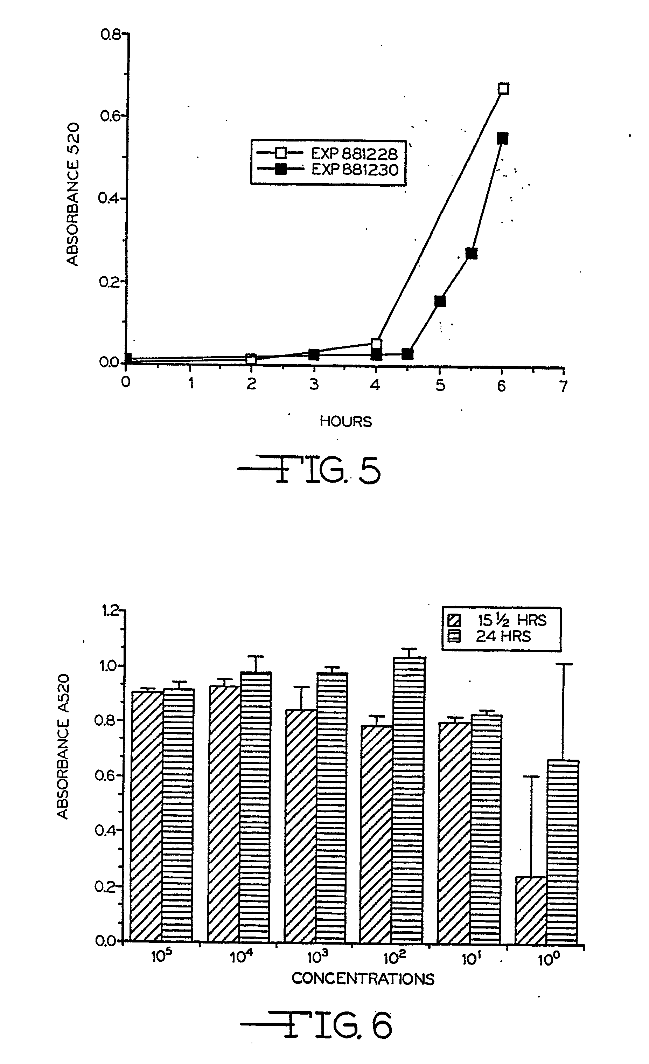

In Fig. 5 the comparative viability assay color response curves for the heat activated spores contained on strips can be readily seen after as short a time period as a five hour incubation.

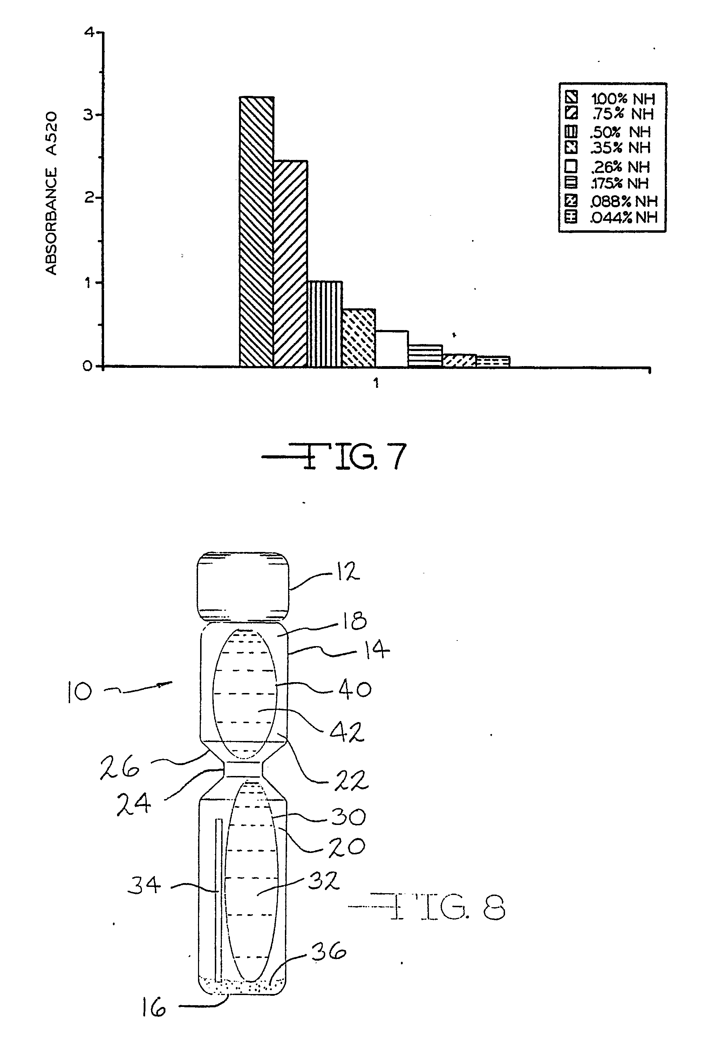

Fig. 6 shows the ninhydrin intensity (blue color development) with paper strips innoculated with decreasing concentrations of Bacillus stearothermophilus spores at both 15.5 and 24 hours. The sensitivity of the spore viability/ninhydrin assay is demonstrated to be $10^0$ (I spore per test) after a 15-24 hour incubation period. The high standard deviation at $10^0$ spores per test is primarily due to a number of the replicate treated tubes actually containing no spores, thereby giving a no color reaction and skewing the absorbance down with accompanying high standard deviations.

Fig. 7 demonstrates that the use of ninhydrin as the indicator agent will work with a concentration as low as about 0.245%. Below that concentration the color development appears not to be as effective in detecting sufficient amino groups to indicate bacterial viability. Above .35% ninhydrin background color development confused positive readings. The data indicated that in the standard assay (Example A or B) an optical density reading of 0.2 or greater will indicate spore viability.

The following specific examples are provided for the purpose of illustrating the present invention using two different container means. The present method determines inadequate or incomplete sterilization of materials or objects so that the biological safety of the materials or objects can be assured. It is understood that these examples serve by way of illustration only and are not intended to limit the invention to the precise material shown nor the application or use thereof.

## EXAMPLE A

The determination of resistance to sterilant was determined as follows:

Spores ($10^5$) contained on filter paper strips enclosed in glassine envelopes, which are commercially available, were autoclaved for the required length of time and then opened and aseptically introduced to tube containing TSB medium for incubation at 55°C for periods of time ranging from 6 to 16 hours. After the incubation time the indicator agent comprising ninhydrin contained in acetone and butanol was added and mixed. The mixture was then incubated at 37°C for 30 minutes. After incubation of the mixture the tubes were evaluated visually for the development of blue color and were quantitated on a spectrophotometer at 520nm.

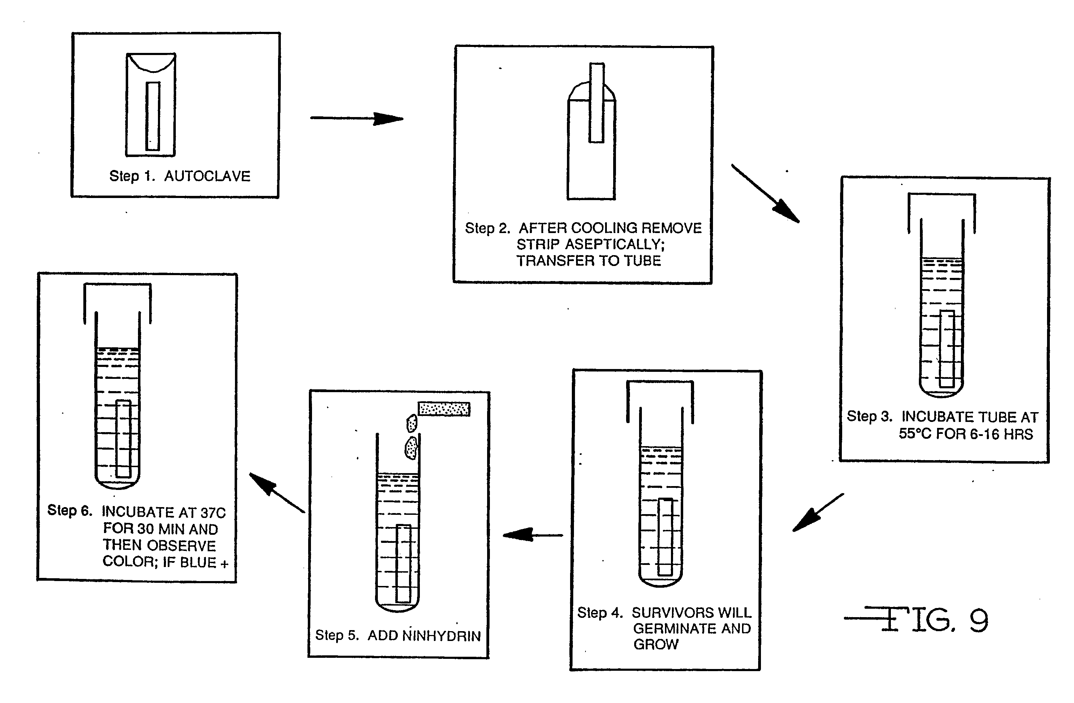

Referring now to Fig. 9, a schematic diagram of one embodiment of steps for carrying out the method for rapidly detecting biological sterility is shown. Step 1: In this embodiment a commercially available glassine envelope containing a cellulose strip with $10^5$ spores is placed in an autoclave along with articles to be sterilized. Step 2: After the sterilization procedure is completed and the articles and spore strips are cooled, the cellulose strip is aseptically removed from the glassine envelope and transferred to a sterile tube containing a nutrient medium comprising a nutrient medium and a protein substrate. Step 3: The sterile tube is incubated at approximately 55°C for a period of time ranging from 6-16 hours. Step 4: During the incubation period any surviving microorganisms will germinate and grow. Step 5: At the end of the incubation period, the indicator agent comprising ninhydrin is added to the test tube. Step 6: After a further incubation period, at approximately 37°C for 30 minutes, the test tube is then observed for any color change. If the color blue is detected then the test is positive.

Example B

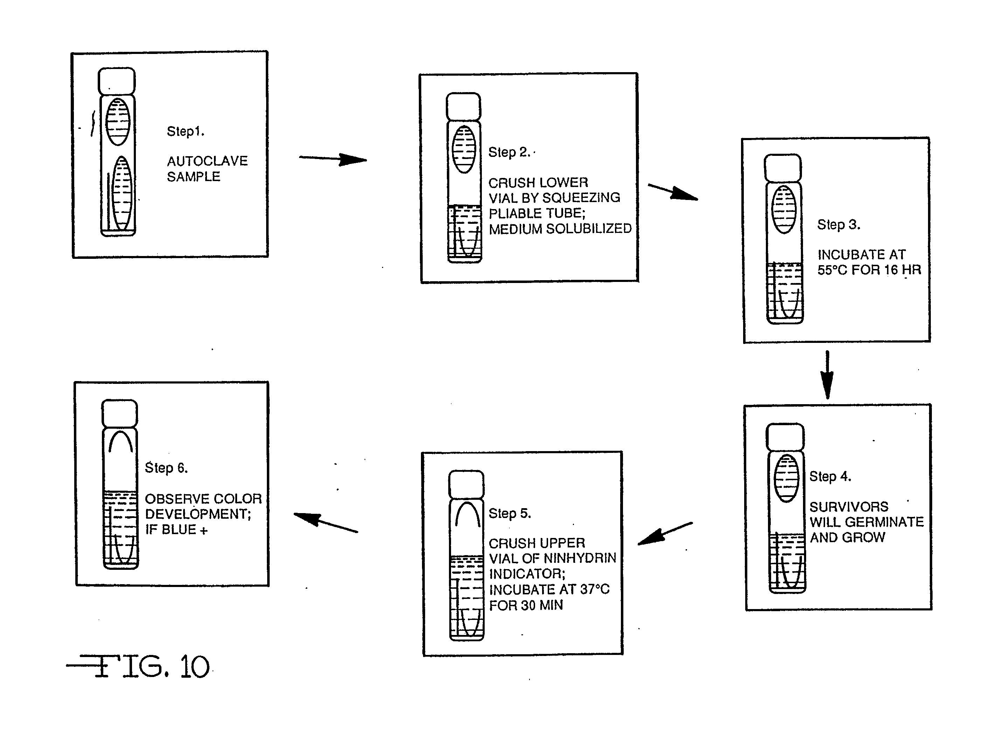

The present invention also provides for the use of a self-contained, crushable apparatus for the rapid determination of sterility. The steps for carrying out the method of this invention using a self-contained, crushable apparatus are shown in the schematic diagram of Fig. 10. The apparatus generally includes a clear pliable or deformable tube having a cap which allows the sterilizing environment to enter the tube during the sterilizing procedure and which prevents comtamination of the contents of the tube after the sterilizing procedure is complete. Various caps (not shown) are commercially available which define, for example, an open pathway into the tube when the cap is in an open position and which aseptically seal the tube when in a closed position. As shown in Fig. 8, the pliable tube contains a cellulose spore strip, a first crushable glass vial containing a dilute sterile buffer, a second crushable vial containing the ninhydrin indicator agent, (.35% ninhydrin in acetone/butanol) and a predetermined quantity of a dessicated protein substrate. Step 1: The apparatus is placed in an autoclave along with the articles to be sterilized. Step 2: Following autoclaving and after the apparatus has cooled sufficiently to be handled safely, a person conducting the sterility test closes the cap, squeezes the clear pliable tube crushing the first glass vial which releases the dilute buffer. The protein substrate is solubilized upon release of the buffer to form a nutrient medium for any surviving microorganisms. Step 3: The apparatus is incubated at approximately 55°C for a period of time ranging from 6 to 16 hours. Step 4: During the incubation period any surviving microorganisms will germinate and grow in the nutrient medium. Step 5: At the end of the incubation period, the pliable tube is again squeezed so that the second crushable vial is broken and the ninhydrin indicator agent is mixed with the nutrient medium. Step 6: After a further period of incubation, approximately 30 minutes at 37°C, the color development is visually determined in comparison to negative controls. Alternatively, color development can be quantitated on a spectrophotometer at 520nm.

Referring now to Fig. 8, a schematic diagram of another embodiment of an apparatus for use in rapidly detecting biological sterility is generally shown. The apparatus 10 comprises a cap 12 and a clear pliable tube 14 having a closed end 16 and an open end 18. The cap 12 matingly engages the open end 18 of the clear pliable tube 14 such that the cap 12 allows the sterilizing environment to enter the tube 14 during the sterilizing procedure and prevents contamination of the contents of the tube after the sterilizing procedure is complete. Various known caps (not shown) are commercially available which define, for example, an open pathway into the tube when the cap is in an open position and which aseptically seal the tube when in a closed position.

The clear pliable tube 14 defines a first or lower chamber 20 and a second or upper chamber 22. The lower chamber 20 is in communication with the upper chamber 22 through an invagination channel 24, such that the invagination channel 24 and the upper chamber 22 define an invagination vial support 26. The lower chamber 20 contains a first crushable glass vial 30 containing a dilute buffer 32. The lower chamber 20 further includes a test sample 34 containing a known quantity of viable sporulated microorganisms distributed on a cellulose strip and a dessicated or powdered nutrient medium 36 comprising, for example, a trypic soy broth medium and a protein substrate. The dessicated nutrient medium 36 rests at the bottom or closed end 16 of the clear pliable tube 14. The use of a powdered culture medium and protein substrate is advantageous in that the shelf life of the apparatus can be greatly extended.

The upper chamber 22 contains a second crushable glass vial 40 containing an indicator agent 42. The crushable vial 40 rests against the invagination vial support 26 such that the crushable vial 40 is held in a position away from the invagination channel 24 so that the sterilizing environment, when the cap 12 is in its first or open position, can circulate within both the upper chamber 22 and the lower chamber 20.

In a preferred embodiment, the invagination channel 24 defines an interior diameter slightly greater than the exterior diameter of the first crushable vial 30 such that the first crushable vial 30 can be positioned within the lower chamber 20 during assembly of the apparatus 10. In a preferred embodiment, the interior diameter of the invagination channel 24 is less than the exterior diameter of the second crushable vial 40 such that the second crushable vial 40 rests within the upper chamber 22 and is held in a position away from the invagination channel 24.

It is to be understood that the lower chamber 20 is in communication with the upper chamber 22 at all times. This can be accomplished by having a series of grooves (not shown) extending longitudinally around the invagination channel 24. Alternatively, the second crushable vial 40 can have a configuration such that while the crushable vial 40 is nestled within the upper chamber 22 and resting against the invagination vial support 26, the crushable vial 40 does not block the invagination channel 24 or prevent communication of the lower chamber 20 with the upper chamber 22.

The present invention has been described with respect to the preferred embodiment. It will be clear to those skilled in the art that modification and/or variations of the disclosed compositions and method can be made without departing from the scope of the invention set forth herein. The invention is defined by the claims as follows.

## Claims

1. A method for determining the effectiveness of a sterilization process comprising the steps of:
   placing a test sample containing a known quantity of viable sporulated microorganisms into a sterilization chamber along with objects to be sterilized;
   removing the test sample from the sterilization chamber along with the objects being sterilized after completion of the sterilization process;
   exposing the test sample to a known quantity of a nutrient medium which supports germination and growth of any remaining viable microorganisms;
   incubating the remaining viable microorganisms for a predetermined period of time to promote the germination and growth of the remaining viable microorganisms;
   exposing any germinating and growing microorganisms to an indicator agent capable of undergoing a detectable change in response to the germination and growth of the remaining viable microorganisms, the indicator agent reacting with end products resulting from protease enzyme reactions produced by the germinating and growing microorganisms; and
   observing whether any detectable change occurs.

2. The method of claim 1 wherein the detection of the end products of the protease enzyme reaction is determined by observing a color reaction produced by the reaction of the indicator agent with the end products.

3. The method of claim 2 wherein the indicator agent undergoes a visible color change.

4. The method of claim 1 wherein the indicator agent comprises a concentration of ninhydrin sufficient to produce a color change when reacted with the end products of protease enzymes produced by the remaining viable germinating and growing microorganisms.

5. The method of claim 4 wherein the concentration of ninhydrin ranges from about 0.2% to about 1.0%.

6. The method of claim 1 wherein the known, quantity of viable sporulated microorganisms ranges from about $10^4$ to about $10^6$ per test sample.

7. The method of claim 1 wherein the known quantity of viable sporulated microorganisms is at least 1 viable sporulated microorganism per test sample.

8. The method of claim 1 wherein the test sample is incubated at about 55°C for a time period ranging from about 6 to 16 hours.

9. The method of claim 1 wherein the viable sporulated microorganisms are selected from the group consisting of Bacillus stearothermophilis or Bacillus subtilis.

10. An apparatus for determining the effectiveness of a sterilization process comprising:
    a clear pliable tube;
    a first crushable vial containing a known quantity of a dilute buffer;
    a second crushable vial containing a known quantity of an indicator agent;
    a test sample containing a known quantity of viable sporulated microorganisms;
    a known quantity of a dessicated nutrient medium; and,
    a closure means capable of allowing a sterilizing environment to enter the pliable tube during the sterilization process and capable of aseptically sealing the pliable tube from the influence of ambient air and possible contamination subsequent to the sterilization process.

11. The apparatus of claim 10 wherein the pliable tube defines a first chamber in communication with a second chamber, the first chamber containing the first crushable vial, the test spore strip sample and the nutrient medium, and the second chamber containing the second crushable vial.

FIG. 1

FIG. 2

FIG. 3

FIG. 4

FIG. 5

FIG. 6

Legend:
- ⊠ 100% NH
- ⊠ .75% NH
- ⊞ .50% NH
- ⊠ .35% NH
- ☐ .26% NH
- ⊟ .175% NH
- ⊠ .088% NH
- ⊞ .044% NH

ABSORBANCE A520

FIG. 7

FIG. 8

Step 1. AUTOCLAVE

Step 2. AFTER COOLING REMOVE STRIP ASEPTICALLY; TRANSFER TO TUBE

Step 3. INCUBATE TUBE AT 55°C FOR 6-16 HRS

Step 4. SURVIVORS WILL GERMINATE AND GROW

Step 5. ADD NINHYDRIN

Step 6. INCUBATE AT 37C FOR 30 MIN AND THEN OBSERVE COLOR; IF BLUE +

FIG. 9

FIG. 10

Step 1.
AUTOCLAVE SAMPLE

Step 2.
CRUSH LOWER VIAL BY SQUEEZING PLIABLE TUBE; MEDIUM SOLUBILIZED

Step 3.
INCUBATE AT 55°C FOR 16 HR

Step 4.
SURVIVORS WILL GERMINATE AND GROW

Step 5.
CRUSH UPPER VIAL OF NINHYDRIN INDICATOR; INCUBATE AT 37°C FOR 30 MIN

Step 6.
OBSERVE COLOR DEVELOPMENT; IF BLUE +

EP 0 433 053 A1

European
Patent Office

EUROPEAN SEARCH
REPORT

Application Number

EP 90 31 3540

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,P | EP-A-0 371 682   (MINNESOTA MINING AND MANUFAC-TURING CO.) <br> * Abstract; claims 1-3,5-7; example 1 * <br> — — — | 1-3,6-9 | C 12 Q <br> 1/22 <br> A 61 L 2/26 |
| A | US-A-4 528 268   (H.W. ANDERSEN) <br> * Claim 1; column 6, lines 48-57 * <br> — — — — — | 1,9-11 | |

TECHNICAL FIELDS
SEARCHED (Int. Cl.5)

C 12 Q
A 61 L

The present search report has been drawn up for all claims

| Place of search | Date of completion of search | Examiner |
|---|---|---|
| The Hague | 07 March 91 | MERLU B.H.A. |

CATEGORY OF CITED DOCUMENTS

X : particularly relevant if taken alone
Y : particularly relevant if combined with another
document of the same catagory
A : technological background
O : non-written disclosure
P : intermediate document ·
T : theory or principle underlying the invention

E : earlier patent document, but published on, or after
the filing date
D : document cited in the application
L : document cited for other reasons

&: member of the same patent family, corresponding
document